# EUROPEAN PATENT APPLICATION

(11) **EP 2 363 099 A1**
(43) Date of publication of application: **07.09.2011**
(21) Application number: 11275039.3
(22) Date of filing: 28.02.2011
(51) Int. Cl.: A61F 2/84

(54) **Medical device delivery system**

(30) Priority: 01.03.2010 US 309166 P; 09.12.2010 US 964154
(71) Applicant: Koven Technology Canada Inc., Winnipeg, Manitoba R3B 1Y6 (CA)
(72) Inventor: Maclean, Ian, Winnipeg Manitoba R3B 1Y6 (CA); Garcia, Enrique, Winnipeg Manitoba R3B 1Y6 (CA); Thebault, Marc Andre, Winnipeg Manitoba R3B 1Y6 (CA); Brooks, Donald, Winnipeg Manitoba R3B 1Y6 (CA); Rayes, Fady, Winnipeg Manitoba R3B 1Y6 (CA)
(74) Representative: Turner, Rhiannon Rosalind

(57) **Abstract**

A medical device delivery system (10) for remotely deploying a stent or similar medical device is disclosed. The medical device delivery system includes a delivery device (11) having a handle (12) engaged to an elongated barrel (14) with a puller arrangement (16) attached to an elongated outer catheter defining a distal end engaged to a catheter tip that collectively encapsulates the medical device. The handle includes a trigger actuator (26) operatively engaged to a drive mechanism with a lead screw that drives the puller arrangement along the barrel. During deployment, the trigger actuator is actuated by the user, which activates the drive mechanism and rotates the lead screw for moving the puller arrangement to automatically deploy the medical device. In manual operation, the user disengages the lead screw from the puller arrangement and manually moves the puller arrangement toward the handle to manually deploy the medical device rather than actuate the trigger actuator.

## Description

### FIELD

The present document relates to a medical device delivery system, and in particular to a medical device delivery system having a delivery device for remotely deploying a stent or other type of medical device.

### BACKGROUND

Medical device delivery systems for remotely deploying a medical device, such as a stent, are known in the art. Such medical device delivery systems can deliver a medical device to a desired location for treatment, and then selectively deploy the medical device in position by actuation of a handle. In operation, these known medical device delivery systems are generally advanced within the body of the patient along a desired vascular pathway or other suitable body passageway until the medical device has reached the desired site for deployment and treatment. During deployment of the medical device, the physician usually holds a proximal hub attached to the inner shaft member of the device in a fixed position with one hand, while also withdrawing the distal hub attached to the outer tubular sheath with the other hand. However, for longer deployment lengths, such a manual deployment operation by the physician requires a certain degree of skill and dexterity to manage the gradual resilient expansion of a medical device as the outer sheath is retracted during deployment. As such, there is a need in the art for a medical device delivery system having a delivery device for automatically and remotely deploying the medical device using only one hand to actuate the handle of the delivery device during the deployment operation.

### SUMMARY

In one embodiment, a delivery apparatus for deploying a medical device includes a handle for remotely deploying the medical device. The handle has a trigger actuator operatively engaged to a drive mechanism for actuating a rotatable lead screw. The handle is engaged to a hollow, elongated barrel defining at least one slot for engaging a puller arrangement. The puller arrangement includes a chariot assembly engaged to the lead screw for moving the puller arrangement in a linear direction along the barrel. The chariot assembly has an automatic chariot engaged to the lead screw for providing linear movement of the chariot assembly by rotation of the lead screw and a manual chariot engaged to the automatic chariot and disengaged from the lead screw for manual actuation of the puller arrangement. A hollow, elongated outer catheter having a proximal end is engaged to the puller arrangement and a distal end thereof is engaged to a catheter tip for collectively encapsulating the medical device prior to deployment. In addition, a hollow, elongated inner catheter is disposed inside the outer catheter with the inner catheter defining a free end engaged to the catheter tip for retaining the catheter tip in a fixed position as the puller arrangement is moved away in a linear direction along the axis of the barrel such that the distal end of the outer catheter disengages the catheter tip and progressively retracts from the medical device during deployment.

The puller arrangement may further include a luer mounted on the chariot assembly and in fluid flow communication with a valve arrangement, which may be in fluid flow communication with the outer catheter. The valve arrangement may be a Y-shaped valve. The lead screw may define a threaded portion engaged to the automatic chariot such that rotation of the lead screw causes the puller arrangement to move in the linear direction along the axis of the barrel. The lead screw may further define a blank portion that prevents movement of the chariot assembly and establishes a termination point. The chariot assembly may further include a pin for coupling together the manual chariot to the automatic chariot. In this case, disengaging the pin from the chariot assembly may disengages the manual chariot from the automatic chariot. The puller arrangement may then manually be moved along the slot of the barrel after the manual chariot is disengaged from the automatic chariot to effectuate deployment of the medical device.

The drive mechanism of the apparatus may include a trigger switch in operative engagement with the trigger actuator and a geared motor having a rotatable output shaft, a drive coupler having one end engaged to the rotatable output shaft and another end thereof being engaged to the lead screw, wherein operation of the drive mechanism causes rotation of the drive coupler for rotating the lead screw. The drive mechanism may further include one or more batteries for providing power to the trigger switch.

The delivery apparatus may further comprise a luer in fluid flow communication with a hollow, elongated hypo tube having an opposing end slideably engaged with the puller arrangement for guiding the puller arrangement along the hypo tube in a linear direction.

The medical device may progressively expand outwardly as the outer sheath progressively retracts from the medical device.

In the delivery apparatus of the invention, the barrel may include at least one protrusion and the handle may be configured to define at least one window for engagement with the at least one protrusion for permitting the barrel to be engaged and disengaged from the handle. The at least one slot may be a pair of opposing slots defined along the longitudinal axis of the barrel. The puller arrangement may then include a puller handle adapted to ride along the at least one slot of the barrel when the puller arrangement is moved away in the linear direction.

In any embodiment of apparatus of the invention, the medical device may be a self-expandable stent.

In the delivery apparatus of the invention, the puller arrangement may further include a Tuohy Borst valve. In this case, the apparatus may further comprise a rigid hypo tube slidably engaged with the puller arrangement for sliding the puller arrangement along the rigid hypo tube, wherein the Tuohy Borst valve provides a means for varying the degree of friction between the puller arrangement and the rigid hypo tube.

The delivery apparatus according to the invention may be for use in a method for automatic remote deployment of a medical device, the method comprising: inserting the catheter tip over a guidewire and through a body passageway of a body for delivery of the medical device to a desired location in the body; and actuating the trigger actuator of the handle in a single-handed action for operating the drive mechanism to rotate the lead screw such that the puller arrangement moves in a linear direction along the slot of the barrel, thereby causing the outer catheter to be progressively retracted and disengaged from the catheter tip to deploy the medical device. The method may further comprise withdrawing the inner catheter with the fixed catheter tip through the medical device after deployment. The medical device may be a stent.

The delivery apparatus according to the invention may be for use in a method for manual remote deployment of a medical device comprising: inserting the catheter tip over a guidewire and through a body passageway of a body for delivery of the medical device to a desired location in the body; and disengaging the manual chariot from the automatic chariot of the chariot assembly such that only the manual chariot is engaged to the puller arrangement; and moving the puller arrangement in a linear direction along the slot of the barrel, thereby causing the outer catheter to be progressively retracted and disengaged from the catheter tip to deploy the medical device. The method may further comprise withdrawing the inner catheter with the fixed catheter tip through the medical device after deployment. The medical device may be a stent.

In another embodiment, a method for automatic remote deployment of a medical device includes the steps of providing a delivery device having a handle for remotely deploying a medical device with the handle including a trigger actuator operatively engaged to a drive mechanism for actuating a rotatable lead screw. A hollow, elongated barrel has a proximal end engaged to the handle with the barrel defining at least one slot for engaging a puller arrangement. The puller arrangement includes a chariot assembly engaged to the lead screw for moving the puller arrangement in a linear direction along the barrel. The chariot assembly includes an automatic chariot engaged to the lead screw for providing linear movement of the chariot assembly along the lead screw and a manual chariot engaged to the automatic chariot and disengaged from the lead screw for manual actuation of the puller arrangement. A hollow, elongated outer catheter having a proximal end engaged to the puller arrangement and a distal end thereof engaged to a catheter tip for collectively encapsulating the medical device prior to deployment. In addition, the hollow, elongated inner catheter is disposed inside the outer catheter with the inner catheter defining a free end engaged to the catheter tip for retaining the catheter tip. Once the delivery device is provided, the user inserts the catheter tip through a body passageway of a body for delivery of the medical device to a desired location of the body. The user then actuates the trigger actuator of the handle in order to rotate the lead screw such that the puller arrangement is moved in a linear direction along the slot of the barrel, thereby causing the outer catheter to be progressively retracted and then disengaged as the medical device is gradually deployed.

In yet another embodiment, a method for manual remote deployment of a medical device includes the steps of providing a delivery device having a handle for remotely deploying a medical device with the handle including a trigger actuator operatively engaged to a drive mechanism for actuating a rotatable lead screw. A hollow, elongated barrel has a proximal end engaged to the handle with the barrel defining at least one slot for engaging a puller arrangement. The puller arrangement includes a chariot assembly engaged to the lead screw for moving the puller arrangement in a linear direction along the barrel. The chariot assembly includes an automatic chariot engaged to the lead screw for providing linear movement of the chariot assembly along the lead screw and a manual chariot engaged to the automatic chariot and disengaged from the lead screw for manual actuation of the puller arrangement. A hollow, elongated outer catheter has a proximal end engaged to the puller arrangement and a distal end thereof engaged to a catheter tip for collectively encapsulating the medical device prior to deployment. In addition, the hollow, elongated inner catheter is disposed inside the outer catheter with the inner catheter defining a free end engaged to the catheter tip for retaining the catheter tip. Once the delivery device is provided, the user inserts the catheter tip through a body passageway of a body for delivery of the medical device to a desired location of the body. Once the medical device is positioned, the user disengages the manual chariot from the automatic chariot of the chariot assembly such that only the manual chariot is engaged to the puller arrangement. The user then moves the puller arrangement in a linear direction along the slot of the barrel, thereby causing the outer catheter to be progressively retracted and disengaged from the catheter tip to gradually deploy the medical device.

Throughout the description and claims of this specification, the words "comprise" and "contain" and variations of the words, for example "comprising" and "comprises", mean "including but not limited to" and do not exclude other components, integers or steps. Throughout the description and claims of this specification, the singular encompasses the plural unless the context otherwise requires. In particular, where the indefinite article is used, the specification is to be understood as contemplating plurality as well as singularity, unless the context requires otherwise.

Preferred features of each aspect of the invention may be as described in connection with any of the other aspects. Other features of the present invention will become apparent from the following examples. Generally speaking, the invention extends to any novel one, or any novel combination, of the features disclosed in this specification (including the accompanying claims and drawings). Thus, features, integers or characteristics described in conjunction with a particular aspect, embodiment or example of the invention are to be understood to be applicable to any other aspect, embodiment or example described herein, unless incompatible therewith. Moreover, unless stated otherwise, any feature disclosed herein may be replaced by an alternative feature serving the same or a similar purpose.

Additional objectives, advantages and novel features will be set forth in the description which follows or will become apparent to those skilled in the art upon examination of the drawings and detailed description which follows.

### BRIEF DESCRIPTION OF THE DRAWINGS

FIG. **1** is an elevated perspective view of a delivery device for a medical device delivery system that remotely deploys a self-expandable stent;

**FIG. 2** is an elevated perspective view of the delivery device illustrating a handle engaged to a barrel of the device;

FIG. **3** is a perspective view of the delivery device showing portions of the device in phantom to illustrate the internal components of the device;

FIG. **4** is a perspective view of the delivery device showing portions of the device in phantom to illustrate the drive mechanism of the device;

FIG. **5** is an enlarged elevated perspective view of the delivery device showing the catheters in a partial cut away view;

FIG. **6** is an enlarged perspective view of the delivery device showing the handle of the device;

FIG. **7** is an enlarged cross-sectional view of the handle taken along line **7-7** of FIG. **6****;**

FIG. **8** is an enlarged cross-sectional view of the barrel for the delivery device;

FIG. **9** is a side view illustrating the internal components of the barrel for the delivery device;

FIG. **10** is an electrical schematic illustration of a drive mechanism for the delivery device;

FIG. **11** is an enlarged perspective view of the delivery device illustrating the engagement of the handle to the barrel of the device;

FIG. **12** is an enlarged cross-sectional view of the delivery device illustrating the operative coupling of the geared motor with the lead screw for remote deployment of the stent;

FIGS. **13-16** illustrate the sequence of progressively moving a puller arrangement during manual deployment of the stent by the delivery device;

FIGS. **17-20** illustrate the sequence of deploying the stent that corresponds to the same deployment sequence shown in FIGS. **13-16****;**

FIG. **21** is an enlarged cross-sectional view illustrating the operative engagement of the lead screw with the drive coupler of the motor; and

FIG. **22** is an enlarged perspective view of the tab used to engage the barrel to the handle of the device.

Corresponding reference characters indicate corresponding elements among the view of the drawings. The headings used in the figures should not be interpreted to limit the scope of the claims.

### DETAILED DESCRIPTION

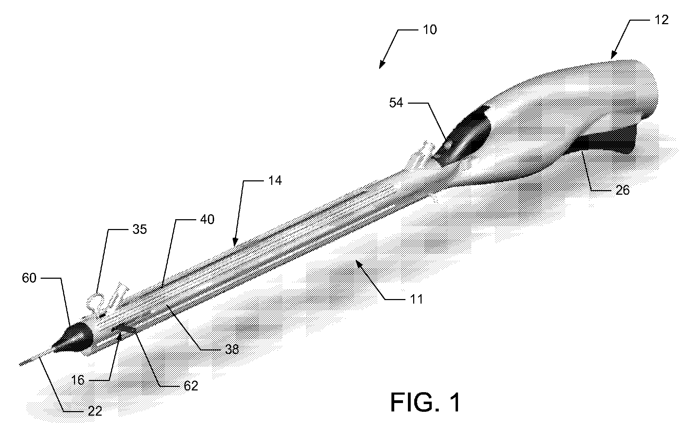

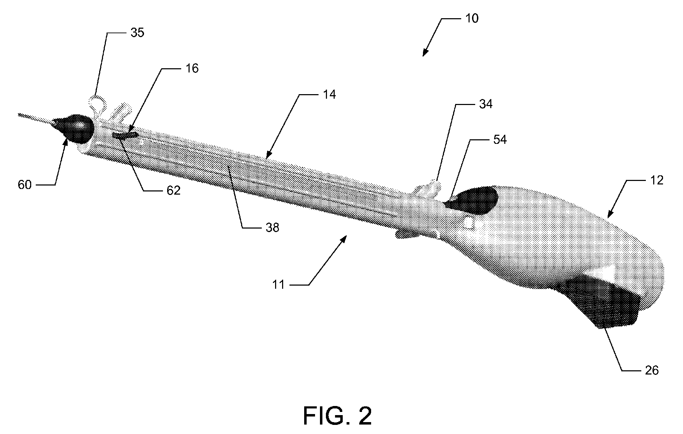

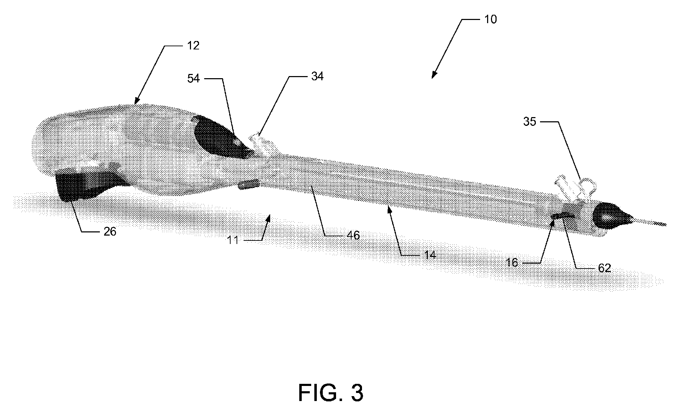

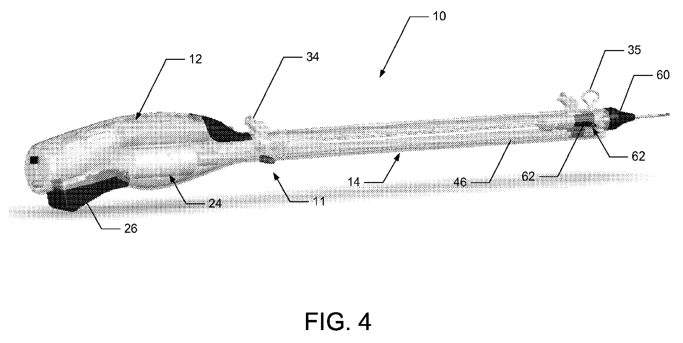

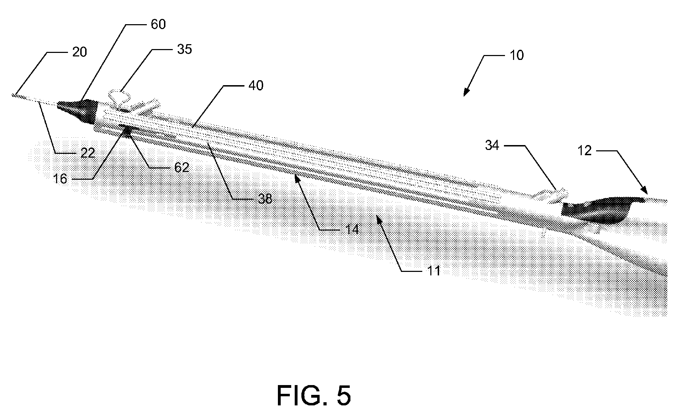

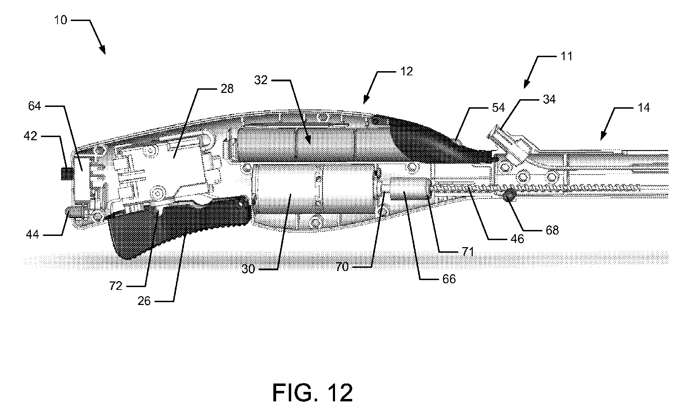

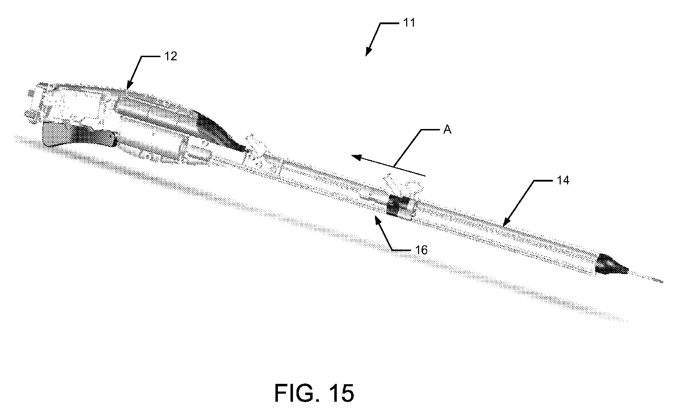

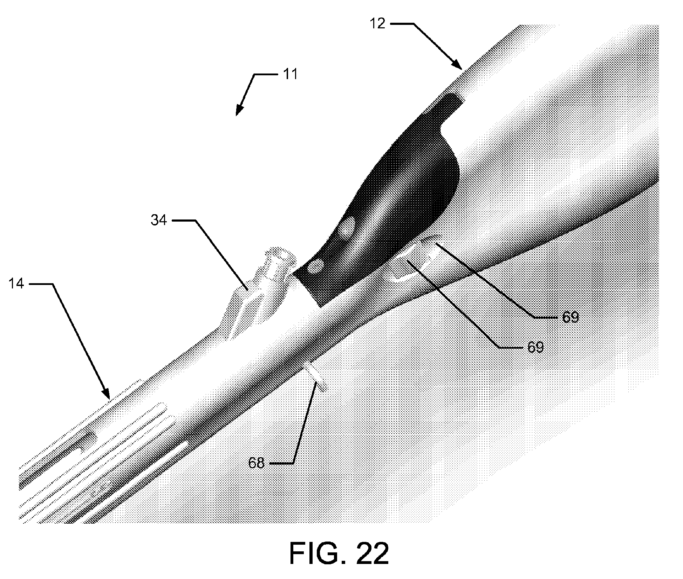

Referring to the drawings, embodiments of a medical device delivery system for remotely deploying a self-expandable stent or other suitable medical device is illustrated and generally indicated as **10** in FIGS. **1-22****.** However, for clarity and convenience, the embodiments of the medical device delivery system **10** discussed herein will only describe a system for deploying self-expandable stents.

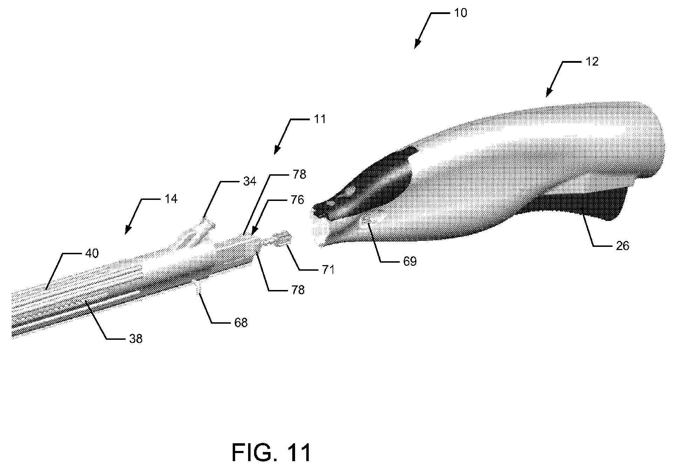

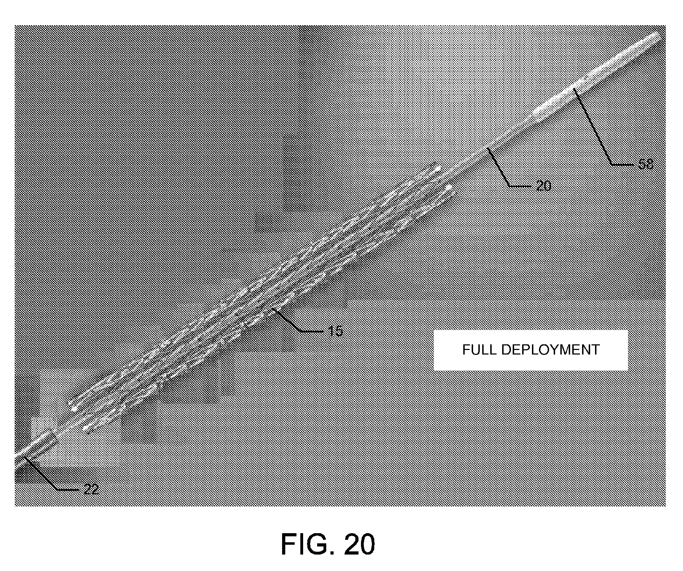

As shown in FIGS. 1-3, an embodiment of the medical device delivery system 10 includes a delivery device 11 having a handle 12 operatively engaged to a barrel 14 for remote deployment of a medical device, such as a stent 15 (FIG. 20), when the user actuates the handle 12 using a single handed action. As shown in FIG. 11, the proximal end of the barrel 14 includes a quick connect tab arrangement 76 adapted to engage the barrel 14 to the distal end of the handle 12. In this way, barrels 14 having different lengths may be engaged to the handle 12. The quick connect tab arrangement 76 has a pair of flexible tabs 78 adapted to engage a respective window 69 defined at the distal end of the handle 12. In particular, each tab 78 is configured to seat and snap into a respective window 69 in order to engage the handle 12 to the barrel 14. In addition, the user may push against the seated tabs 76 from each respective window 69 to disengage the barrel 14 from the handle 12. In this manner, different types of barrels 14 may be engaged to the handle 12.

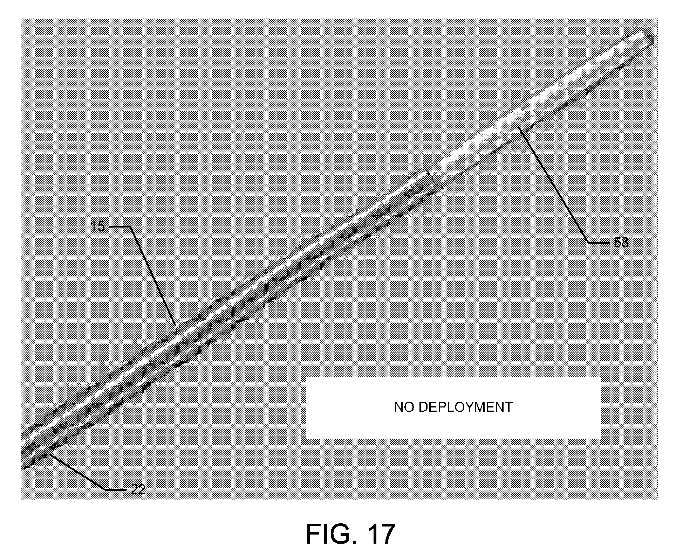

Prior to deployment as illustrated in FIG. 17, the stent 15 is maintained in a constrained position by an elongated, flexible outer catheter 22 that surrounds the stent 15 until retracted by actuation of the delivery device 11. The outer catheter 22 is engaged to the delivery device 11 through a puller arrangement 16 that is adapted to move along the longitudinal axis of the barrel 14 as the outer catheter 22 is being gradually retracted by the user operating the delivery device 11 in either manual or automatic modes of operation as shall be described in greater detail below. As further shown in FIG. 1, a strain relief 60 is attached to the free end of the barrel 14 to provide structural support to the distal end of the barrel 14, especially to stresses that may be applied to the barrel 14 by the outer catheter 22, and to also minimize the risk of the outer catheter 22 from becoming kinked. As used herein, the term "proximal" shall mean that portion of the delivery device 11 closest to the handle 12, while the term "distal" shall mean that portion of the delivery device 11 farthest away from the handle 12.

Referring back to FIGS. 1-4, barrel 14 may define an elongated upper slot 40 and a pair of opposing elongated side slots 38 for allowing movement of the puller arrangement 16 along the longitudinal axis of the barrel 14. The handle 12 may include a trigger actuator 26 operatively associated to the puller arrangement 16 through a drive mechanism 24 such that actuation of the drive mechanism 24 by the trigger actuator 26 causes the puller arrangement 16 to automatically move in the proximal direction along the longitudinal axis of the barrel 14. This proximal action of the puller arrangement 16 causes the gradual deployment of the constrained stent 15 as the outer catheter 22 progressively retracts and then disengages from the stent 15 to allow deployment of the stent 15. The puller arrangement 16 is operatively engaged to a lead screw 46 for automatically driving the puller arrangement 16 along the longitudinal axis of the barrel 14 by the drive mechanism 24 upon actuation of the trigger actuator 26. In particular, rotation of the lead screw 46 by the drive mechanism 24 causes the puller arrangement 16 to be driven in the proximal direction towards the handle 12 for withdrawing the outer catheter 22 and remotely deploying the stent 15.

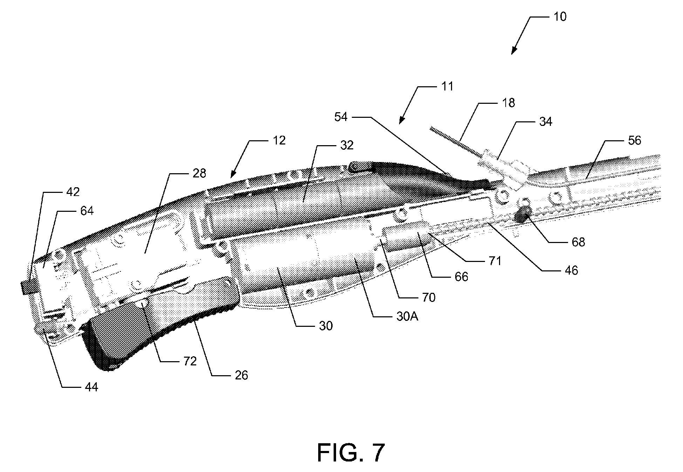

As shown in FIG. 7, trigger actuator 26 actuates the drive mechanism 24 to automatically drive the puller arrangement 16 along slots 38 and 40 of the barrel 14 which causes the outer catheter 22 to progressively release and deploy the stent 15. In the automatic operation of the delivery device 11, the drive mechanism 24 rotates the lead screw 46 and causes the puller arrangement 16 to be automatically driven along the longitudinal axis of the barrel 14 in the proximal direction. The drive mechanism 24 includes a trigger switch 28 that is operatively engaged to the trigger actuator 26, which actuates a geared motor 30 for causing rotation of the lead screw 46. The geared motor 30 is supplied with power by a plurality of batteries 32, such as lithium batteries, encased inside the body of the handle 12. In operation, the user may manually engage the trigger actuator 26 in an upward movement, which causes the trigger switch 28 to actuate the geared motor 30 that progressively retracts the outer catheter 22 and deploys the stent 15 as the puller arrangement 16 is concurrently moved in the proximal direction by rotation of the lead screw 46. In one embodiment, actuation of the trigger actuator 26 produces a variable speed rotation by the geared motor 30 depending on the degree the trigger actuator 26 is depressed.

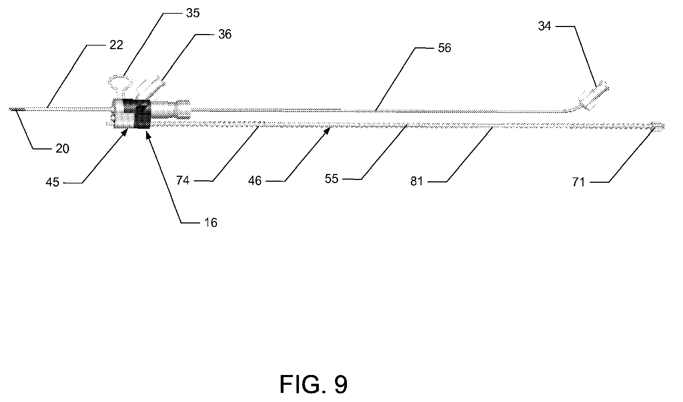

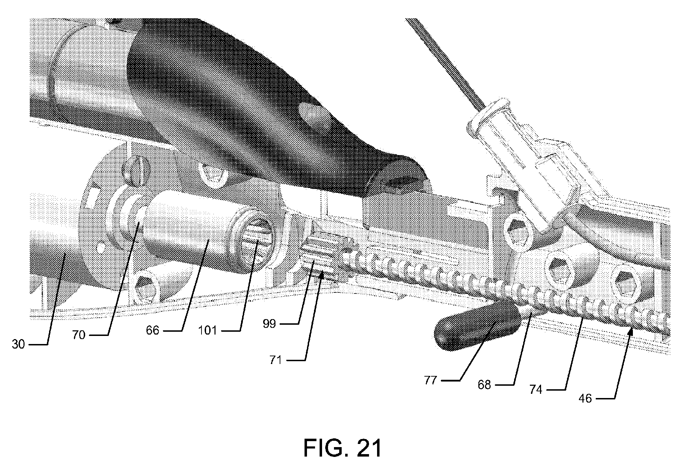

Referring to FIG. 9, the lead screw 46 may define a threaded portion 55 having a plurality of successively defined external threads 74 adapted to engage and drive the puller arrangement 16 along the external threads 74 and a blank portion 81 having a flat surface defined along the lead screw 46. The blank portion 81 of the lead screw 46 ensures that the stent 15 is fully deployed since the absence of any external threads 74 at the blank portion 81 of the lead screw 46 causes the puller arrangement 16 to stop at a termination point 80 where the outer catheter 22 has been withdrawn sufficiently to fully release the stent 15. As further shown in FIG. 21, the proximal end of the lead screw 46 includes a male coupler 71 defining longitudinal threads 99 configured for engagement with the internal threads 101 defined within the cavity of a drive coupler 66. The drive coupler 66 is operatively engaged to the geared drive 30A such that the lead screw 46 is rotated by the motor 30. As shown in FIG. 7, the motor 30 is attached to a geared drive 30A, which reduces the output speed and increases the output torque of the motor 30. The geared drive 30A has an output shaft 70 engaged to the driver coupler 66. In this arrangement, the driver coupler 66 couples the output shaft 70 of the geared drive 30A to the male coupler 71 of the lead screw 46, which is detachably engaged to the driver coupler 66. Rotation of the output shaft 70 by the geared drive 30A concurrently rotates the driver coupler 66 in order to rotate the lead screw 46 for driving the puller arrangement 16.

As shown, the handle 14 includes a power button 42 operatively engaged to a switch component 64 for providing power to the delivery device 11 and a power indicator 44, such as a light emitting diode (LED), for visually indicating the power status of the device 11. The trigger switch 28 is operatively engaged to the trigger actuator 26 through an actuator component 72, for example a piston, such that actuating or depressing of the trigger actuator 26 causes the geared motor 30 to be made operational by the trigger switch 28.

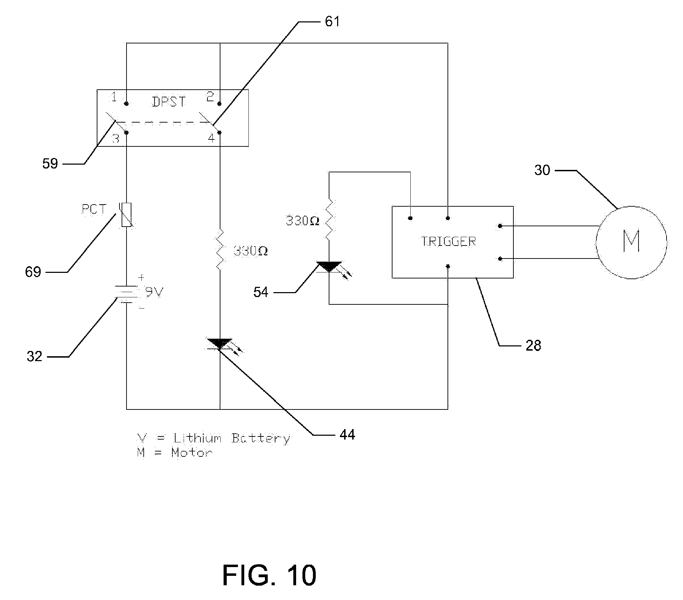

As illustrated in the electrical schematic illustrated in FIGS. 7 and 10, the actuation of the power button 42 triggers the switch component 64 that includes a first switch 59 and a second switch 61. In particular, triggering the switch component 64 causes the first switch 59 to close between terminals 1 and 3 as well as the second switch 61 to close between terminals 2 and 4 to permit the power from the batteries 32 to energize the delivery device 11 and illuminate the power indicator 44 to indicate a power on condition. In one embodiment, a 330Ω resistor is in series with the power indicator 44 and power indicator 54. Moreover, a positive temperature coefficient ("PTC") component 69 may be in a series with the batteries 32 for preventing an over current condition. Once the first and second switches 59 and 61 are closed upon activation of the power button 42, the trigger switch 28 may be activated by actuation of the trigger actuator 26. Actuation of the trigger actuator 26 causes the power indicator 54 to be illuminated for visually indicating that the motor 30 is operational and that automatic deployment of the stent 15 is occurring.

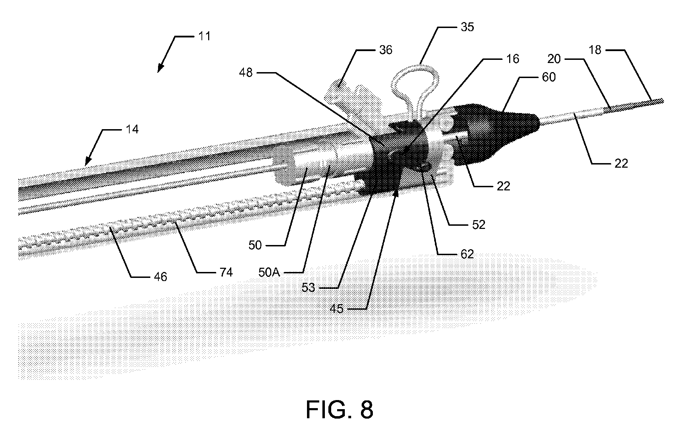

Referring to FIG. 8, the puller arrangement 16 provides a means for progressively deploying the stent 15 in either manual or automatic operation of the delivery device 11. The puller arrangement 16 includes a Tuohy Borst valve 50 engaged to a Y connector 48 mounted on a chariot assembly 45 for either manually or automatically deploying the stent 15 by the delivery device 11. The chariot assembly 45 includes an automatic chariot 52 that is operatively engaged and driven along the threaded portion 55 of the lead screw 46 when actuated by the drive mechanism 24. As noted above, the lead screw 46 defines a plurality of external threads 74 adapted to engage internal threads defined within the channel of the automatic chariot 52 such that rotation of the lead screw 46 causes the automatic chariot 52 and the puller arrangement 16 to travel in a linear proximal direction along the lead screw 46.

Referring to FIG. 9, the smooth portion 81 of the lead screw 46 defines a portion of the lead screw 46 having no external threads 74 that would permit operative engagement with the automatic chariot 52. As such, the smooth portion 81 defines the termination point 80 along the lead screw 46 that prevents any further travel of the puller arrangement 16 since the internal threads defined within the automatic chariot 52 are no longer engaged to the external threads 74 of the lead screw 46. As shown in FIGS. 7 and 21, a locking pin 68 is inserted through barrel 14 and between the external threads 74 of the lead screw 46 to prevent rotation of the external threads 74 and maintain alignment of the lead screw 46 during shipment of the delivery device 11. As shown, the locking pin 68 maintains the lead screw 46 in alignment with the drive coupler 66 of the handle 12. This structural arrangement ensures that when the barrel 14 is properly aligned and connected to the handle 12 by the quick connect tab arrangement 76, the male coupler 71 fits correctly into the drive coupler 66. The locking pin 68 also provides a safety function that the user must first release prior to using the delivery device 11. As shown, the locking pin 68 may include a cover 77 that encapsulates a portion of the pin 68 and is removed prior to disengaging the pin 68 from the lead screw 46. In one embodiment, the user disengages the locking pin 68 and withdraws the locking pin 68 from it engagement with the external threads 74 to permit rotation of the lead screw 46.

As noted above, the lead screw 46 includes a threaded portion 74 that is engaged to the puller arrangement 16 through the automatic chariot 52. The automatic chariot 52 includes a channel (not shown) having internal threads adapted to engage the external threads 74 defined by lead screw 46 in order to drive the puller arrangement 16 in a linear direction towards the handle 12 when actuated by the trigger actuator 26 by rotation of the lead screw 46. During manual operation of the delivery device 11, the manual chariot 53 is not directly engaged to the external threads 74 of the lead screw 46, thereby allowing the user to disengage the manual chariot 53 from the chariot assembly 45 and manually move the puller arrangement 16 along the lead screw 46 without actuating the trigger actuator 26 to accomplish this action. To effect the decoupling of the automatic chariot 52 from the manual chariot 53 for permitting manual operation, a pin 35 is pulled from the chariot assembly 45 to disengage the manual chariot 53 from the automatic chariot 52 and allow the manual chariot 53 to freely ride over the lead screw 46 as the puller arrangement 16 is moved in the proximal direction by drawing back on the puller handle 62. This manual action of the puller arrangement 16 also acts to withdraw the outer catheter 22 and deploy the stent 15.

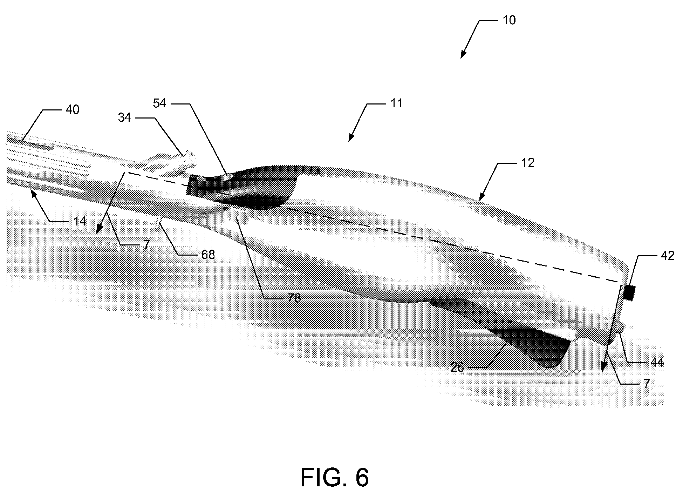

As shown in FIGS. 6 and 7, the barrel 12 also includes a luer 34 located near the handle 12 having a lumen adapted to receive a syringe (not shown) for flushing a guidewire lumen (not shown) with fluid. The guidewire lumen is the inner lumen of the hollow, rigid hypo tube 56 attached between the luer 34 and strain relief 60. During travel of the puller arrangement 16 along the lead screw 46, the puller arrangement 16 is also concurrently traveling over the hollow, rigid tube 56, which acts as a guide. In one embodiment, the Tuohy Borst valve 50 may be a haemostatic seal to prevent fluid loss from the outer catheter 22 and includes a rotatable threaded cap 50A that may be rotated to either increase or decrease the amount of resistance generated between the hypo tube 56 and the valve 50 as the puller arrangement 16 travels along the hypo tube 56.

The outer catheter 22 has a distal end for encapsulating the stent 15 prior to deployment and a proximal end attached to the puller arrangement 16. As discussed above, the outer catheter 22 provides a means for remotely deploying the stent 15 by operation of the handle 12 as the outer catheter 22 is disengaged from the catheter tip 58 and progressively retracted from the stent 15 directly due to the gradual proximal travel of the puller arrangement 16 along the longitudinal axis of the barrel 14. As the outer catheter 22 is progressively retracted due to the linear movement of the puller arrangement 16, the encapsulated stent 15 is allowed to progressively deploy and expand within the bodily lumen as shall be discussed in greater detail below.

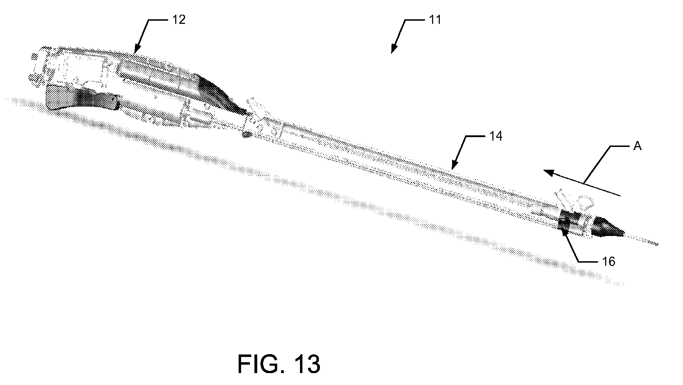

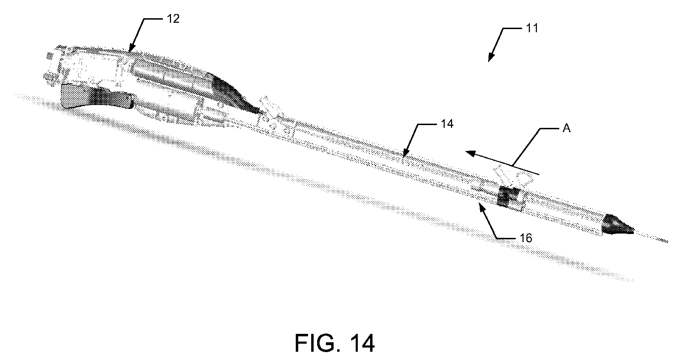

Referring to FIGS. 13-16, the manual operation for deploying the stent 15 without having to actuate the trigger actuator 26 is illustrated. This manual means of deploying the stent 15 by manually driving the puller arrangement 16 in the proximal direction toward the handle 12 may be used when the drive mechanism 24 becomes inoperable, thereby preventing automatic deployment of the stent 15 by actuation of the trigger actuator 26. Prior to deployment of the stent 15, the user disengages the pin 35 that connects the automatic chariot 52 from the manual chariot 53. The user then directly engages the puller handle 62 and manually drives the puller arrangement 16 in the proximal direction A toward the handle 12 using the puller handle 62 as shown in FIG. 13. As illustrated in FIGS. 14-16, the user continues to draw back on the puller arrangement 16 in the proximal direction A until the puller handle 62 reaches termination point 80 along the barrel 14, thereby preventing any further proximal movement of the puller arrangement 16. As the puller arrangement 16 is drawn toward the handle 12, the stent 15 is progressively deployed as the distal end of the outer catheter 22 retracts in the proximal direction such that the stent 15 gradually expands outwardly to a full deployment position when the puller arrangement 16 reaches termination point 80.

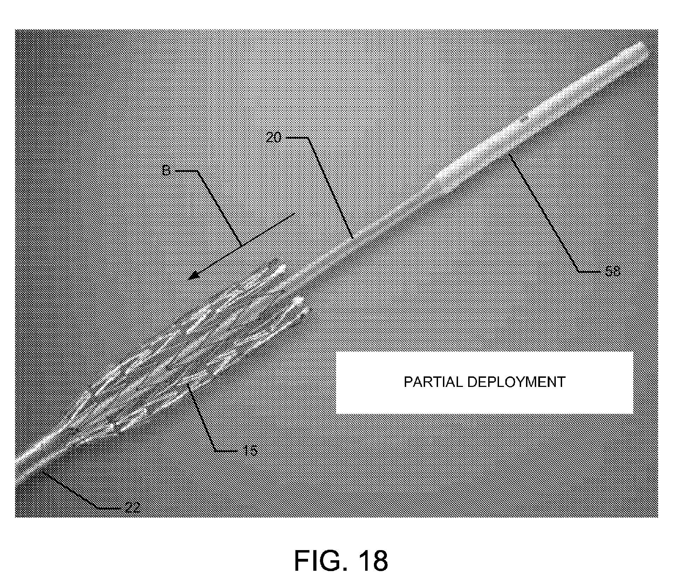

As shown in FIGS. 17-20, the deployment of the stent 15 will be described in greater detail. Prior to the puller arrangement 16 being either manually or automatically drawn back as shown in FIG. 13 to initiate deployment, the catheter tip 58 is engaged with the distal end of the outer catheter 22 to encapsulate the stent 15 within the outer catheter 22 with the tip 58 closing off the distal end of the outer catheter 22. The interior of the catheter tip 58 is fixed to an inner catheter 20 such that the catheter tip 58 is maintained at a fixed distance relative to the barrel 14. When the outer catheter 22 is progressively retracted, the catheter 22 becomes disengaged from the catheter tip 58 in the fixed position and the stent 15 is allowed to gradually expand and deploy from the outer catheter 22 into a partial deployment position as illustrated in FIG. 18.

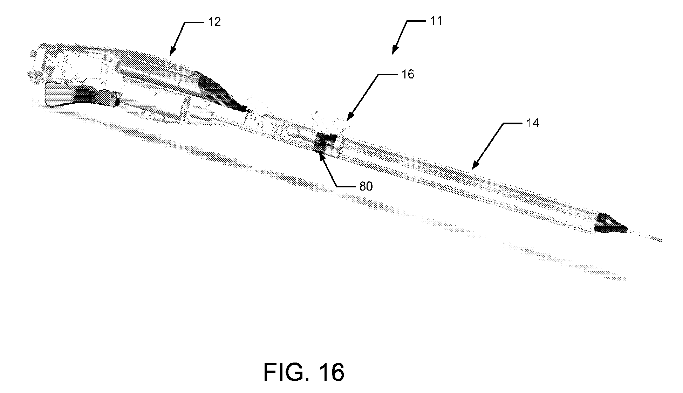

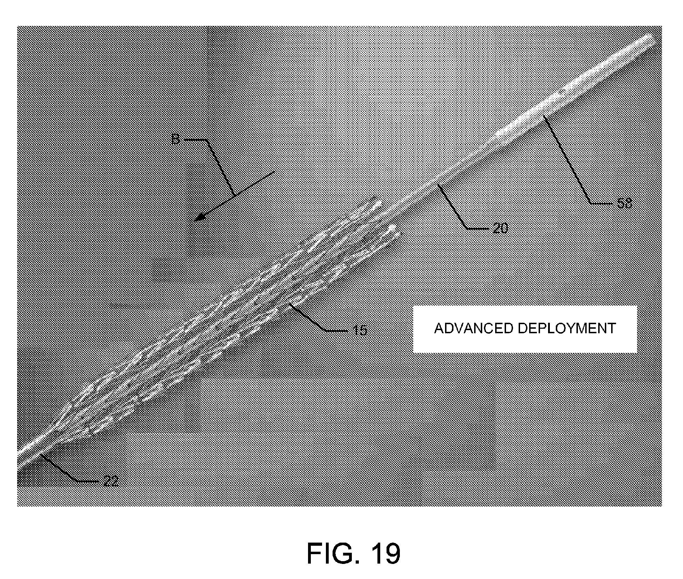

Once the stent 15 is partially deployed, the proximal movement in direction A of the puller arrangement 16 continues to draw the distal end of the outer catheter 22 toward the handle 12 such that the stent 15 continues to be gradually released from the outer catheter 22 and expand outwardly as illustrated in FIG. 19. After the puller arrangement 16 has reached termination point 80 (FIG. 16) along barrel 14, the stent 15 has been completely released from the outer catheter 22 and is fully deployed as shown in FIG. 20, thereby completing the remote deployment of the stent 15. Once the deployment of the stent 15 has been completed, the inner catheter 20, with the fixed catheter tip 58 will be withdrawn though the stent 15 as the outer catheter 22 is withdrawn from the patient thus completing the deployment operation.

It should be understood from the foregoing that, while particular embodiments have been illustrated and described, various modifications can be made thereto without inventive skill, as will be apparent to those skilled in the art.

## Claims

1. A delivery apparatus for deploying a medical device comprising:
a handle for remotely deploying a medical device, the handle including
a trigger actuator operatively engaged to a drive mechanism for
actuating a rotatable lead screw,
a hollow, elongated barrel having a proximal end engaged to the
handle, the barrel defining at least one slot for engaging a puller arrangement, the puller arrangement comprising:
a chariot assembly engaged to the lead screw for moving the puller arrangement in a linear direction along the barrel, the chariot assembly including an automatic chariot engaged to the lead screw for providing linear movement of the chariot assembly by rotation of the lead screw, and a manual chariot engaged to the automatic chariot and not engaged to the lead screw for manual actuation of the puller arrangement;
a hollow, elongated outer catheter having a proximal end engaged to
the puller arrangement and a distal end thereof engaged to a catheter tip for collectively encapsulating the medical device prior to deployment, and
a hollow, elongated inner catheter disposed inside the outer catheter,
the inner catheter defining a free end engaged to the catheter tip for retaining the catheter tip in a fixed position as the puller arrangement is moved away in a linear direction along the axis of the barrel such that the distal end of the outer catheter disengages from the catheter tip and progressively retracts from the medical device during deployment.

2. The delivery apparatus of claim 1, wherein the puller arrangement further including a luer mounted on the chariot assembly and in fluid flow communication with a valve arrangement.

3. The delivery apparatus of claim 2, wherein the valve arrangement is in fluid flow communication with the outer catheter.

4. The delivery apparatus of any preceding claim, wherein the lead screw defines a threaded portion engaged to the automatic chariot such that rotation of the lead screw causes the puller arrangement to move in the linear direction along the axis of the barrel.

5. The delivery apparatus of claim 4, wherein the lead screw further defines a blank portion that prevents movement of the chariot assembly and establishes a termination point.

6. The delivery apparatus of claim 5, wherein the chariot assembly further includes a pin for coupling together the manual chariot to the automatic chariot.

7. The delivery apparatus of claim 6, wherein disengaging the pin from the chariot assembly disengages the manual chariot from the automatic chariot.

8. The delivery apparatus of claim 7, wherein the puller arrangement is manually moved along the slot of the barrel after the manual chariot is disengaged from the automatic chariot to effectuate deployment of the medical device.

9. The delivery apparatus of any preceding claim, wherein the drive mechanism includes a trigger switch in operative engagement with the trigger actuator and a geared motor having a rotatable output shaft, a drive coupler having one end engaged to the rotatable output shaft and another end thereof being engaged to the lead screw, wherein operation of the drive mechanism causes rotation of the drive coupler for rotating the lead screw.

10. The delivery apparatus of any preceding claim, further comprising:
a luer in fluid flow communication with a hollow, elongated hypo tube
having an opposing end slideably engaged with the puller arrangement for guiding the puller arrangement along the hypo tube in a linear direction.

11. The delivery apparatus of any preceding claim, wherein the medical device progressively expands outwardly as the outer sheath progressively retracts from the medical device.

12. The delivery apparatus of any preceding claim, wherein the barrel includes at least one protrusion and the handle is configured to define at least one window for engagement with the at least one protrusion for permitting the barrel to be engaged and disengaged from the handle.

13. The delivery apparatus of any preceding claim, wherein the puller arrangement further includes a Tuohy Borst valve.

14. The delivery apparatus of claim 13, further comprising a rigid hypo tube slidably engaged with the puller arrangement for sliding the puller arrangement along the rigid hypo tube, wherein the Tuohy Borst valve provides a means for varying the degree of friction between the puller arrangement and the rigid hypo tube.

15. The delivery apparatus according to any preceding claim for use in a method for automatic remote deployment of a medical device comprising:
inserting the catheter tip over a guidewire and through a body
passageway of a body for delivery of the medical device to a desired location in the body; and either
a) actuating the trigger actuator of the handle in a single-handed action for operating the drive mechanism to rotate the lead screw such that the puller arrangement moves in a linear direction along the slot of the barrel, thereby causing the outer catheter to be progressively retracted and disengaged from the catheter tip to deploy the medical device; or
b) disengaging the manual chariot from the automatic chariot of the chariot assembly such that only the manual chariot is engaged to the puller arrangement, and moving the puller arrangement in a linear direction along the slot of the barrel, thereby causing the outer catheter to be progressively retracted and disengaged from the catheter tip to deploy the medical device.
